Europäisches Patentamt

European Patent Office

Office européen des brevets

(11) Veröffentlichungsnummer: **0 141 410**

**A2**

(12) **EUROPÄISCHE PATENTANMELDUNG**

(21) Anmeldenummer: **84113212.9**

(22) Anmeldetag: **02.11.84**

(51) Int. Cl.⁴: **A 23 D 5/00**
**A 61 K 7/00, A 61 K 47/00**
**A 21 D 2/02, A 21 D 2/16**
**C 10 M 123/02**

(30) Priorität: **10.11.83 DE 3340680**

(43) Veröffentlichungstag der Anmeldung:
**15.05.85 Patentblatt 85/20**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Heine, Christian, Dr.**
**Habichtstrasse 4**
**D-4019 Monheim(DE)**

(72) Erfinder: **Wüst, Reinhold**
**Jahnstrasse 33**
**D-4044 Kaarst(DE)**

(54) Verfahren zur Erhöhung der Viskosität von Ölen.

㊼ Ein synergistischer Effekt auf das Viskositätsverhalten von Ölen wird durch Zusatz einer Kombination aus 0,1-10 Gew.% eines hochschmelzenden Fettsäureglycerids von gesättigten Fettsäuren ($C_{14-24}$) mit einem Schmelzpunkt von mehr als 50°C (A) und 0,2-10 Gew.% einer hochdispersen Kieselsäure (B) im Verhältnis A:B= 20:80 bis 80:20 erzielt. Eine deutliche Viskositätserhöhung läßt sich mit relativ geringen Mengen von 0,1-1,0 Gew.% (A) und 0,2-2 Gew.% (B) erreichen; zur Erzielung pastöser Konsistenz sind Zusatzmengen von 1 Gew.% (A) und 2 Gew.% (B) erforderlich (Mengenverhältnis A:B= 25:75 bis 50:50).

EP 0 141 410 A2

0141410

HENKEL KGaA
ZR-FE/Patente
Dr. Bz/Sr

4000 Düsseldorf, den 25. 10. 1983

Patentanmeldung

D 6785 EP

"Verfahren zur Erhöhung der Viskosität von Ölen"

Es ist bekannt, Viskositätserhöhungen von Ölen durch Zusätze von feinteiligen Feststoffen zu erreichen. Insbesondere ist hierzu hochdisperse Kieselsäure geeignet. Andererseits führen auch Zusätze an hochschmelzenden Fettstoffen, z. B. gehärteten Glyceriden mit höheren Kettenlängen zu dem gleichen Ziel.

Zusätze der genannten Stoffe können darüber hinaus gegebenenfalls Thixotropie-Erscheinungen hervorrufen. Letzteres bedeutet, daß die Öle unter Scherbeanspruchung ihre Viskosität vermindern, bei ruhigem Stehen aber wieder in den höherviskosen Zustand übergehen.

Es wurde nun überraschenderweise ein signifikanter synergistischer Effekt auf das Viskositätsverhalten von Ölen gefunden, dergestalt, daß sich hochdisperse Kieselsäure und hochschmelzende Fettstoffe in ihrer Wirksamkeit in unerwartet starkem Maße gegenseitig verstärken.

Gegenstand der Erfindung ist demgemäß ein Verfahren zur Erhöhung der Viskosität bzw. zur Verdickung von Ölen, dadurch gekennzeichnet, daß man diesen 0,1 - 10 Gew.-% eines hochschmelzenden Fettsäureglycerids von gesättigten Fettsäuren der Kettenlänge $C_{14}$ - $C_{24}$ und einem Schmelzpunkt von mehr als 50 $^\circ$C (A), und 0,2 - 10 Gew.-% einer hochdispersen Kieselsäure (B) im Verhältnis von (A) : (B) wie 20 : 80 bis 80 : 20, in einer Gesamtmenge von 0,3 - 20 Gew.-% zusetzt. Die Prozentangaben beziehen sich auf das gesamte Gemisch, also auf das verdickte Öl.

Unter dem Begriff "Öle" im Sinne der Erfindung werden wasserunlösliche, bei Raumtemperatur flüssige organische Verbindungen mit niedrigem Dampfdruck verstanden, deren gemeinsames Merkmal die ölartige physikalische Konsistenz ist. Die Öle weisen eine Viskosität von wenigstens 40mPas und ein Molgewicht von wenigstens 140 auf. Insbesondere kommen hier in erster Linie natürliche oder synthetische Glyceride höherer Fettsäuren der Kettenlängen $C_6$ - $C_{24}$ in Betracht, wobei die Fettsäuren der Kettenlängen $C_{12}$ - $C_{24}$ eine oder mehrere Doppelbindungen aufweisen. Ferner sind zu nennen höhere Fettsäuren oder Fettalkohole der Kettenlängen $C_6$ - $C_{24}$ und deren Ester und Ether, soweit sie ölartige Konsistenz aufweisen, sowie Mineralöle.

Geeignete hochschmelzende Fettsäureglyceride (A) sind die Mono-, Di- und insbesondere Triglyceride der Palmitin-Stearin-, Oxystearin- und Behensäure, gegebenenfalls mit Anteilen an höheren Fettsäuren bis $C_{24}$, die Schmelzpunkte von 50 bis 90 $^{\circ}$C aufweisen. Beispiele hierfür sind gehärtete pflanzliche oder tierische Öle, wie Rizinusöl, Talg, Schweineschmalz, Palmöl sowie Glycerinmonostearat oder Glycerinmonobehenat.

Als bekanntes Beispiel für hochdisperse Kieselsäure (B) ist Aerosil $^{(R)}$ (Degussa) zu nennen.

Das erfindungsgemäße Verfahren zur Erhöhung der Viskosität ist für zahlreiche Anwendungsgebiete von erheblicher technischer Bedeutung:

So lassen sich z. B. kosmetische und pharmazeutische Öle auf Basis physiologisch verträglicher, flüssiger Ölkomponenten in ihrer Viskosität beliebig anheben.

...

Solche verträglichen flüssigen Ölkomponenten sind z. B.
pflanzliche und tierische Öle, z. B. Triglyceride und
Fettalkoholester auf Basis von Fettsäuren mit einem
höheren Anteil an ungesättigten Fettsäuren mit der Kettenlänge $C_{12}$ - $C_{22}$, vor allem Ölsäure, Linolsäure, Erucasäure,
z. B. Olivenöl, Rüböl, Palmöl, Sojaöl, Spermöl. Weitere
geeignete kosmetisch-pharmazeutische Ölkomponenten sind
flüssige, mittelkettige Fettsäuretriglyceride aus Fettsäuren der Kettenlänge $C_8$ - $C_{10}$, Fettsäureester höherer
Fettalkohole wie z. B. Oleyloleat, Fettsäureester niederer
Alkohole wie z. B. Isopropylmyristat, Fettsäureester verzweigtkettiger Alkohole wie z. B. 2-Ethylhexylstearat,
2-Hexyl-decyl-palmitat, flüssige ungesättigte Fettalkohole
wie z. B. Oleylalkohol, verzweigte Alkohole mit 12 - 36
C-Atomen wie z. B. 2-Hexyl-decanol, 2-Octyl-dodecanol,
schließlich auch Fettalkoholpolyglycolether, z. B. das
Anlagerungsprodukt von 15 Mol Ethylenoxid an Oleylalkohol,
oder das Anlagerungsprodukt von 10 Mol Ethylenoxid und
20 Mol Propylenoxid an Cetyl-Stearylalkohol, Fettsäurepolyglycolester, Fettsäureester von Ethylenoxid- und
Propylenoxidanlagerungsprodukten an Fettalkohole, Glycole
oder Glycerin sowie auch flüssige Paraffinöle.

Ein anderes Anwendungsgebiet für das erfindungsgemäße
Verfahren ist das der Speiseöle, der Trägeröle für Nahrungsmittelzusätze, z. B. für Gewürze, und der Trennöle für
Back- und Süßwaren. Speiseöle und Trägeröle für Gewürze
und Aromen werden auf Basis flüssiger pflanzlicher und
tierischer Fettsäuretriglyceridöle, z. B. Sojaöl, Sonnenblumenöl, Palmöl, Rapsöl, Olivenöl, Kokosöl und Gemischen
solcher und anderer bekannter Nahrungsmittelöle hergestellt. Auch ölartige Mono- und Diglyceride, z. B.
Glycerinmonooleat und Glycerindioleat, die als Emulgatoren
in der Nahrungsmittelindustrie Verwendung finden, lassen
sich nach dem erfindungsgemäßen Verfahren verdicken.

                                                    ...

Trennöle für Back- und Süßwaren lassen sich nach dem erfindungsgemäßen Verfahren ebenfalls verdicken. Bei diesen Produkten führen bereits relativ geringe Zusätze von 0,1 - 1 Gew.-% an hochschmelzendem Triglycerid (A) und 0,2 - 2,0 Gew.-% an hochdisperser Kieselsäure (B) zu einer erheblichen Erhöhung der Haftwirkung an metallischen Oberflächen, insbesonder bei hohen Temperaturen. Geeignete flüssige Öle sind z. B. flüssige, ungesättigte Triglyceride wie die als Speiseöl geeigneten natürlichen pflanzlichen und tierischen Öle, synthetische ungesättigte Fettsäuretriglyceride und flüssige, ungesättigte Fettsäureester höherer Fettalkohole wie z. B. Spermölfiltrat, Cetyloleat, Oleyloleat, Oleylpalmitat und Gemische dieser Öle. Die nach dem erfindungsgemäßen Verfahren verdickten Öle lassen sich problemlos z. B. mit Hilfe von Sprühpistolen auf die Backformen aufbringen, ohne daß die engen Düsen verstopfen.

Schmiermittel auf Basis flüssiger Mineralöle und/oder flüssiger synthetischer Esteröle lassen sich nach dem erfindungsgemäßen Verfahren beliebig stark bis zur Konsistenz von Schmierfett verdicken. Geeignete synthetische Esteröle sind z. B. die Ester mehrwertiger Alkohole wie Pentaerythrit, Neopentylglycol, Trimethylolpropan, Glycerin mit linearen oder verzweigten Alkancarbonsäuren, bevorzugt mit 5 - 15 C-Atomen, die Ester von Dicarbonsäuren wie Adipinsäure, Azelainsäure, Sebacinsäure, Phthalsäure mit gesättigten, linearen oder verzweigten Alkanolen, bevorzugt mit 4 - 12 C-Atomen, Komplexester aus mehrwertigen Alkoholen, Alkancarbonsäuren und Dicarbonsäuren der genannten Art sowie Fettsäurepolyglycolester und Triethanolaminfettsäureester.

...

Eine signifikante Erhöhung der Viskosität der Öle läßt sich bereits - je nach Art des Öles - mit relativ geringen Mengen von jeweils 0,1 - 1,0 Gew.-% (A) und 0,2 - 2 Gew.-% (B) erreichen. Zur Erzielung pastöser Konsistenzen sind in der Regel Zusatzmengen von wenigstens 1,0 Gew.-% (A) und 2,0 Gew.-% (B), insgesamt etwa 3 - 10,0 Gew.-% (A) und (B) erforderlich. Im einzelnen hängen die erforderlichen Zusatzmengen jedoch von der physikalischen und chemischen Eigenart der Komponenten (A) und (B), wie auch von derjenigen der zu verdickenden Öle ab. Die Bestandteile (A) und (B) können den Ölen einzeln in beliebiger Reihenfolge zugesetzt werden, wobei das Verhältnis (A) : (B), vorzugsweise 25 : 75 bis 50 : 50 Gew.-% ist. Es ist zweckmäßig, zunächst dem erwärmten Öl unter intensivem Rühren das Fettsäureglycid (A), und anschließend die Kieselsäure-Menge (B) zuzugeben und dann die Mischung unter fortgesetztem intensiven Rühren schnell auf Raumtemperatur abzukühlen.

Zur Herstellung nur wenig verdickter Öle, bei welchen es mehr auf die Verbesserung der Haftfähigkeit oder der Dispergier- und Tragefähigkeit als auf die Viskositätserhöhung ankommt, empfiehlt es sich, zunächst ein höherviskoses bis pastöses Konzentrat des hochschmelzenden Triglycerids (A) und der hochdispersen Kieselsäure (B) in dem Öl, dessen Viskosität, Haftfähigkeit oder Tragefähigkeit erhöht werden soll, herzustellen und dieses dann mit dem flüssigen Öl weiter zu verdünnen.
Ein solches Konzentrat enthält bevorzugt 1 - 10 Gew.-% des hochschmelzenden Triglycerids und 2 - 10 Gew.-% der hochdispersen Kieselsäure.

...

Zur Herstellung eines Trennöls für Backwaren würde man ein solches Konzentrat auf der Basis eines der vorgenannten, für Backtrennöle geeigneten flüssigen Öls herstellen. Das für die Anwendung als Backtrennöl geeignete Produkt stellt man aus dem Konzentrat dadurch her, daß man es ohne Wärmezufuhr mit weiterem flüssigen Öl verdünnt, bis der Gehalt an hochschmelzendem Triglycerid (1) in einem Bereich von 0,1 - 1 Gew.-% und der Gehalt an hochdisperser Kieselsäure (B) in einem Bereich von 0,2 - 2 Gew.-%des fertigen Trennöls liegt.

Durch die erfindungsgemäße Maßnahme erreicht man eine außerordentliche Viskositätssteigerung bei den verschiedenartigsten Ölen, wie sie mit einer gleichen Menge eines einzigen der Bestandteile nicht erzielt werden kann. Dabei lassen sich gegebenenfalls auch gelartige oder pastöse Verdickungen erreichen, die auf Grund ihrer thixotropen Eigenschaften durch Rühren oder dergleichen leicht zu verflüssigen sind, was für eine bequeme Handhabung und Anwendung der Produkte von erheblicher Bedeutung sein kann.

Die erfindungsgemäß verdickten Öle weisen darüber hinaus ein verbessertes Haftvermögen an festen Oberflächen, insbesondere bei erhöhten Temperaturen, sowie ein verbessertes Dispergier- und Tragevermögen für dispergierte Zusatzmittel auf. Die folgenden Beispiele sollen den Gegenstand der Erfindung näher erläutern ohne ihn hierauf zu beschränken.

...

## Beispiel 1

Die nachfolgende Tabelle zeigt die Viskositätserhöhung von Rüböl bzw. Mineralöl durch Zusatz von insgesamt 6 Gew.-% verschiedener hochschmelzender Fettsäureglyceride (A) und Aerosil [(R)] (B) in verschiedenen Mengenverhältnissen. Der synergistische Effekt der Kombinationen von (A) und (B) ist klar zu erkennen.

Die Messung der Viskosität erfolgte im Brookfield-Viskosimeter, Typ RVT, bei 20 °C/20 UPM.

0141410

## Viskositätserhöhung von Ölen

| Ölsorte | Rüböl | | | | | | | | | | | | | Mineralöl | | |
|---|---|---|---|---|---|---|---|---|---|---|---|---|---|---|---|---|
| Versuch-Nr. | a | b | c | d | e | f | g | h | i | j | k | l | m | n | o | p |
| **Zusätze in %** | | | | | | | | | | | | | | | | |
| hochdisperse Kieselsäure (Aerosil 200 (R)) | 0 | 6 | 4,5 | 3,9 | 3,6 | 3 | 1,5 | 0 | 3,9 | 3,9 | 3,9 | 3,9 | 3,9 | 0 | 6 | 3,9 |
| gehärtetes Rizinusöl | | | 1,5 | 2,1 | 2,4 | 3 | 4,5 | 6 | | | | | | | | |
| gehärteter Talg | | | | | | | | | 2,1 | | | | | | | |
| gehärtetes Schweineschmalz | | | | | | | | | | 2,1 | | | | | | |
| gehärtetes Palmöl | | | | | | | | | | | 2,1 | | | | | 2,1 |
| Glycerin-Monostearat | | | | | | | | | | | | 2,1 | | | | |
| Glycerin-Monobehenat | | | | | | | | | | | | | 2,1 | | | |
| Viskosität (m Pas) (20 °C, Brookfield-Viskosimeter RVT, 20 Upm) | 100 | 3900 | 26500 | 28500 | 26000 | 22000 | 4400 | 1300 | 26500 | 25000 | 52000 | 11000 | 34000 | 400 | 16000 | 66000 |
| Konsistenz | fl. | di-fl | past. | past. | past | past | past di-fl | fl | past | past | past | past dü-fl | past | fl | past | past |

fl = flüssig
di-fl = dickflüssig
past = pastös
dü-fl = dünnflüssig

0141410
HENKEL KGaA
ZR-FE/Patente

## Beispiel 2

### a) Speiseöl, pastös

1ooo g Rüböl wurden auf $90^{\circ}$C erwärmt, sodann unter Rühren 21 g aufgeschmolzenes durchgehärtetes Rizinusöl (FP $84^{\circ}$) und 39 g Aerosil 2oo$^{(R)}$ eingetragen. Die Mischung wurde unter intenivem Rühren indirekt mit Eiswasser auf $20^{\circ}$C abgekühlt. Nach 24-stündiger Lagerung bei Raumtemperatur wies das Öl eine pastöse Konsistenz auf. Die Viskosität, gemessen im Brookfield-Viskosimeter (Type RVT bei $20^{\circ}$C, 2o UpM), betrug 26.ooo mPas.

### b) Speiseöl, dickflüssig

Wie in Beispiel 2a angegeben, wurden in 1ooo g Rüböl

1o,5 g aufgeschmolzenes durchgehärtetes Rizinusöl (FP $84^{\circ}$) und 19,5 g Aerosil 2oo$^{(R)}$ eingetragen.

Nach 24-stündiger Lagerung bei Raumtemperatur wies das Öl eine dickflüssige Konsistenz und eine Viskosität von 75oo mPas auf.

### c) Speiseöl, flüssig

Wie in Beispiel 2a angegeben, wurden in 1ooo g Rüböl

2,1 g aufgeschmolzenes durchgehärtetes Rizinusöl (FP $84^{\circ}$) und 3,9 g Aerosil 2oo$^{(R)}$ eingetragen.

Nach 24-stündiger Lagerung bei Raumtemperatur wies das Öl eine erhöhte Viskosität von 36o mPas auf.

...

0141410

## Beispiel 3

### a) Mineralöl-Produkt, pastös

1ooo g Mineralöl wurden unter Zusatz von 21 g durchgehärtetem Palmöl (FP 55°) und

39 g Aerosil 2oo$^{(R)}$ in einen Rührbehälter gegeben und auf 9o°C erwärmt. Nachdem die Produkt gelöst waren, wurde unter intensivem Rühren schnell auf 2o°C abgekühlt.

Nach 24 Stunden Lagerung bei Raumtemperatur wies das Öl eine pastöse Konsistenz auf. Die Viskosität (Messung wie in Beispiel 1) betrug 66.ooo mPas. Das Ausgangsmineralöl hatte eine Viskosität von 4oo mPas.

### b) Mineralöl-Produkt, dickflüssig

Wie in Beispiel 3a angegeben, wurden in 1ooo g Mineralöl

7.o g durchgehärtetes Palmöl und

13,o g Aerosil 2oo$^{(R)}$ eingetragen.

Nach 24 Stunden Lagerung bei Raumtemperatur wies das Öl eine dickflüssige Konsistenz und eine Viskosität von 128oo mPas auf.

## Beispiel 4

### a) Hautfunktionsöl mit einem Gehalt an tierischem Gewebe- bzw. Organextrakt, pastös

855 g einer Mischung aus höherem ungesättigtem Alkohol-$C_{16}$-$C_{18}$, Fettsäureisopropylester-$C_{12}$-$C_{14}$ und mittelkettigem Fettsäuretriglycerid-$C_8$-$C_{10}$ wurden in einen Rührbehälter gegeben. Danach wurden

25 g Rizinusöl, durchgehärtet und

45 g Aerosil 2oo$^{(R)}$ in die Mischung eingebracht und auf 9o°C erwärmt, bis eine klare ölige Lösung entstanden war. Dann wurde unter intensivem Rühren schnell auf 2o°C gekühlt und

5 g nativ-lösliches Collagen, und

25 g Weizenkeimöl in die Mischung eingebracht.

Nach einer Lagerung von 24 Stunden bei Raumtemperatur besaß das Hautfunktionsöl eine pastöse Konsistenz. Die Viskosität (Messung wie in Beispiel 1) betrug 1o5oo mPas.

Ohne Zusatz von gehärtetem Rizinusöl und Aerosil war das Hautfunktionsöl dünnflüssig und besaß eine Viskosität von nur 5o mPas.

b) Hautfunktionsöl mit einem Gehalt an tierischem Gewebe- bzw. Organextrakt, dickflüssig

Wie in Beispiel 4a angegeben, wurden in

855 g einer Mischung aus höherem ungesättigtem Alkohol-$C_{16}$-$C_{18}$, Fettsäureisopropylester-$C_{12}$-$C_{14}$ und mittelkettigem Fettsäuretriglycerid-$C_8$-$C_{1o}$

14 g Rizinusöl, durchgehärtet und

26 g Aerosil 2oo$^{(R)}$

5 g tierischer Gewebe- bzw. Organextrakt und

25 g Weizenkeimöl eingetragen.

Nach einer Lagerung von 24 Stunden bei Raumtemperatur besaß das Hautfunktionsöl eine dickflüssige Konsistenz und wies eine Viskosität von 2500 mPas auf.

...

0141410
HENKEL KGaA
ZR-FE/Patente

## Beispiel 5

### a) Ölpflegebad mit Rückfettung, pastös

830 g Oleylalkoholpolyglykolether (7 EO) wurden in einen Rührbehälter gegeben. Unter Rühren wurden

25 g Rizinusöl, durchgehärtet und

45 g Aerosil 2oo[R] zugesetzt. Die Mischung wurde auf $90^{\circ}C$ erwärmt und nach Lösen der Produkte unter intensivem Rühren rasch auf $2o^{\circ}C$ abgekühlt. Zum Schluß wurden

1oo g Parfüm unter Rühren zugegeben.

Nach einer Lagerung von 24 Stunden bei Raumtemperatur wies das Ölpflegebad eine pastöse Konsistenz auf. Die Viskosität (Messung wie in Beispiel 1) betrug 72.ooo mPas.

Das Ölpflegebad ohne Zusatz von gehärtetem Rizinusöl und Aerosil war dünnflüssig und besaß eine Viskosität von nur 1oo mPas.

### b) Ölpflegebad mit Rückfettung, dickflüssig

Wie in Beispiel 5a angegeben, wurden in

830 g Oleylalkoholpolyglykolether (7 EO)

17,5 g Rizinusöl, durchgehärtet und

32,5 g Aerosil 2oo[R]

1oo g Parfüm eingetragen.

Nach einer Lagerung von 24 Stunden bei Raumtemperatur wies das Ölpflegebad eine dickflüssige Konsistenz und eine Viskosität von 79oo mPas auf.

## Beispiel 6

## Kosmetisches Öl

1000 g Decyloleat oder Oleyloleat bzw. ein Gemisch von beiden wurden auf 90 °C erwärmt, sodann unter Rühren

21 g aufgeschmolzenes durchgehärtetes Rizinusöl (FP 84 °) und

39 g Aerosil 200 ® eingetragen. Die Mischung wurde unter intensivem Rühren indirekt mit Eiswasser auf 20 °C abgekühlt. Nach 24-stündiger Lagerung bei Raumtemperatur wies das Öl eine pastöse Konsistenz und eine Viskosität von 32.000 mPas auf. (Messung wie im Beispiel 1).

Das Ausgangsprodukt war dünnflüssig und wies eine Viskosiät von 95 mPas auf.

## Beispiel 7

### a) Speiseöl, pastös

1000 g Sojaöl wurden auf 90 °C erwärmt, sodann unter Rühren

26 g aufgeschmolzenes, gehärtetes Schweineschmalz und
49 g Aerosil 200 (R) eingetragen. Die Mischung wurde unter intensivem Rühren indirekt mit Eiswasser auf 20 °C abgekühlt. Nach 24-stündiger Lagerung bei Raumtemperatur wies das Öl eine pastöse Konsistenz auf. Die Viskosität betrug 29.000 mPas. (Messung wie in Beispiel 1).

## Beispiel 8

### a) Speiseöl, pastös

1000 g Olivenöl wurden auf 90 °C erwärmt, sodann unter Rühren

.21 g aufgeschmolzenes Glycerinmonostearat (FP 67°) und

39 g Aerosil 200[R] eingetragen. Die Mischung wurde unter intensivem Rühren indirekt mit Eiswasser auf 20 °C abgekühlt. Nach 24-stündiger Lagerung bei Raumtemperatur wies das Öl eine pastöse Konsistenz auf. Die Viskosität betrug 18.000 mPas. Messung wie im Beispiel 1).

## Beispiel 9

### a) Speiseöl, pastös

1000 g Sonnenblumenöl wurden auf 90 °C erwärmt, sodann unter Rühren

. 26 g aufgeschmolzenes, gehärtetes Palmöl (FP 55° und 49 g Aerosil 200[R] eingetragen. Die Mischung wurde unter intensivem Rühren indirekt mit Eiswasser auf 20 °C abgekühlt. Nach 24-stündiger Lagerung bei Raumtemperatur wies das Öl eine pastöse Konsistenz auf. Die Viskosität betrug 28.000 mPas. (Messung wie in Beispiel 1).

**0141410**

Beispiel 1o

Backtrennöl

Eine Mischung aus

> 9o,o g Sojaöl-(Raffinat) und
> 4,o g Sojalecithin
> 2,1 g gehärtetem Palmöl und
> 3,9 g Aerosil 2oo ®

wurde auf 9o°C unter Rühren erwärmt.

Die Mischung wurde unter Rühren indirekt mit Eiswasser auf 2o°C abgekühlt. Diese Mischung wurde zu

> 9oo g Sojaöl-Raffinat

unter Rühren bis zur homogenen Vermischung gegeben. Es wurde ein flüssiges Backtrennöl erhalten, dessen Viskosität bei 2o°C 148 mPas betrug.

Die Gesamtmischung wies ohne den Zusatz von gehärtetem Palmöl und Aerosil 2oo eine Viskosität bei 2o°C von 1o5 mPas auf.

Das Backtrennöl wurde mit einer Sprühpistole auf die Backformen aufgedüst.

## Patentansprüche

1. Verfahren zur Erhöhung der Viskosität bzw. zur Verdickung von Ölen, dadurch gekennzeichnet, daß man diesen 0,1 - 10 Gew.-% eines hochschmelzenden Fettsäureglycerids von gesättigten Fettsäuren der Kettenlängen $C_{14}$ - $C_{24}$ und einem Schmelzpunkt von mehr als 50 °C (A), und 0,2 - 10 Gew.-% einer hochdispersen Kieselsäure (B), bezogen auf das gesamte Gemisch, im Verhältnis von (A) : (B) wie 20 : 80 bis 80 : 20, zusetzt.

2. Verfahren nach Anspruch 1, dadurch gekennzeichnet, daß man zur Erzielung einer hohen Viskosität 1 - 10 Gew.-% des hochschmelzenden Fettsäureglycerids (A) und 2,0 - 10 Gew.-% hochdisperse Kieselsäure (B) im Verhältnis von (A) : (B) wie 25 : 75 bis 50 : 50, zusetzt.

3. Verfahren nach Anspruch 1, dadurch gekennzeichnet, daß man zur Erteilung einer erhöhten Viskosität 0,1 - 1 Gew.-% des hochschmelzenden Fettsäureglycerids (A) und 0,2 - 2 Gew.-% hochdisperse Kieselsäure (B) im Verhältnis von (A) : (B) wie 25 : 75 bis 50 : 50, zusetzt.

4. Verfahren nach Anspruch 3, dadurch gekennzeichnet, daß man zunächst 1 - 10 Gew.-% des hochschmelzenden Fettsäureglycerids (A) und 2 - 10 Gew.-% hochdisperse Kieselsäure (B) zusetzt und dann mit dem flüssigen Öl verdünnt, so daß ein Gehalt von 0,1 - 1 Gew.-% (A) und 0,2 - 2 Gew.-% (B) erreicht wird.

...

5. Verfahren nach Anspruch 1 bis 4, worin die hochschmelzenden Fettsäureglyceride (A) Mono-, Di- und
Triglyceride der Palmitin-, Stearin-, Oxystearin-
und Behensäure mit Schmelzpunkten von 50 - 90 $^{\circ}$C sind.

6. Verfahren nach Anspruch 1 bis 5, worin die hochdisperse Kieselsäure Aerosil [R] (Degussa) ist.

7. Verfahren nach Anspruch 1 - 6 zur Erhöhung der Viskosität bzw. zur Verdickung von pflanzlichen oder
tierischen Ölen, synthetischen flüssigen Fettsäure-
mono-, di- und Triglyceriden mit höheren Anteilen
ungesättigter Fettsäuren mit der Kettenlänge $C_{12}$ -
$C_{22}$ und flüssiger, ungesättigter Fettsäureester
ungesättigter Fettalkohole.

8. Kosmetische und pharmazeutische Öle auf Basis verträglicher flüssiger Ölkomponenten, dadurch gekennzeichnet, daß sie nach einem der Verfahren gemäß
Anspruch 1 - 6 in ihrer Viskosität angehoben sind.

9. Schmiermittel auf Basis von flüssigen Mineralölen
oder flüssigen synthetischen Esterölen dadurch gekennzeichnet, daß sie nach einem Verfahren gemäß Anspruch
1 - 6 in ihrer Viskosität angehoben sind.

10. Speiseöle auf Basis pflanzlicher oder tierischer
flüssiger Fettsäuretriglyceride, dadurch gekennzeichnet, daß sie nach einem der Verfahren gemäß Anspruch
1 - 6 in ihrer Viskosität angehoben sind.

...

0141410
HENKEL KGaA
ZR-FE/Patente

11. Trennöl für Backwaren, dadurch gekennzeichnet, daß es aus

97 - 99,7 Gew.-% eines flüssigen ungesättigten Triglyceridöls und/oder eines ungesättigten Fettsäureesters höherer Fettalkohole

0,1 - 1 Gew.-% eines hochschmelzenden Fettsäureglycerids gesättigter Fettsäuren der Kettenlänge $C_{14}$ - $C_{24}$ mit einem Schmelzpunkt von mehr als 50°C und

0,2 - 2 Gew.-% einer hochdispersen Kieselsäure

besteht.